# EUROPEAN PATENT SPECIFICATION

(11) **EP 3 406 703 B1**
(45) Date of publication and mention of the grant of the patent: **22.12.2021**
(21) Application number: 17461633.4
(22) Date of filing: 22.11.2017
(51) Int. Cl.: C12M 1/12, B01L 3/00, C12M 1/42, C12M 1/34

(54) **COMPARTMENT CONTAINER TO PREPARE BIOFILM FOR ANALYSIS WITH SCANNING ELECTRON MICROSCOPY (SEM)**
ABTEILBEHÄLTER ZUR GEWINNUNG VON BIOFILM ZUR ANALYSE MITTELS ELEKTRONENMIKROSKOPIE (SEM)
RÉCIPIENT COMPARTIMENTS POUR L'OBTENTION D'UN BIOFILM POUR L'ANALYSE AU MICROSCOPE ÉLECTRONIQUE SEM

(30) Priority: 22.05.2017 PL 42167317
(43) Date of publication of application: 28.11.2018
(73) Proprietor: Slaski Uniwerytet Medyczny, 40-055 Katowice (PL)
(72) Inventor: Dziedzic, Arkadiusz, 41-902 Bytom (PL); Wojtyczka, Robert, 40-115 Katowice (PL); Kubina, Robert, 40-507 Katowice (PL); Tanasiewicz, Marta, 41-902 Bytom (PL); Kujawski, Wojciech, 87-100 Torun (PL)
(74) Representative: Wroblewski, Marcin Jan

(56) References cited:
- US-A1- 2011 236 769
- US-A1- 2012 190 591
- US-A1- 2013 177 916
- JO PHILIPS ET AL: "Biofilm Formation by Clostridium ljungdahlii Is Induced by Sodium Chloride Stress: Experimental Evaluation and Transcriptome Analysis", PLOS ONE, vol. 12, no. 1, 24 January 2017 (2017-01-24), page e0170406, XP055498374, DOI: 10.1371/journal.pone.0170406
- Bok Yung Kim ET AL: "Development of an Escherichia coli Biofilm Model on Transwell", Korean J Clin Lab Sci, vol. 44, no. 3 1 September 2012 (2012-09-01), pages 112-117, XP55497992, Retrieved from the Internet: URL:1 [retrieved on 2018-08-07]

## Description

The invention is a culture medium compartment container for a microbiological biofilm production in *in vitro* conditions due to a need for an appropriate environment to grow the microorganisms, composing a biofilm studied with scanning electron microscopy (SEM). The divided two-compartment container with a separated carrier allows for a damage-free preparation of biological biofilm on a conductive carbon matrix for use in SEM analysis. The carbon matrix conducting electrons protects the biofilm sample from a damage caused by the SEM-emitted electron stream.

One of the factors determining the composition and structure of the prepared biofilm is a growth medium. Contrary to classic optical microscopy, SEM measurements apply a high-energy electron emitter, which may produce considerable damage in organic samples (biofilm). Because of this, it is important to ensure that the carrier of the organic material for microbiological studies during SEM measurements is a good electron conductor, which prevents energy from accumulating on the sample surface and its negative effect on biofilm structure.

American patent no. US 7871791 B2 covers preparation of biofilm for visualisation with microscopy technology. Publications of international patent applications no. U.S. Patent 6,051,423; U.S. Patent 6,326,190; U.S. Patent 6,410,256; U.S. Patent 6,596,505; U.S. Patent 6,599,696; U.S. Patent 6,599,714 no. US 4,974,243 cover the MBEC High-throughput (HTP) Assay (Innovotech) system for microbiological breeding, which uses broth medium to assess bacteria sensitivity and establishes MIC values.

American patent no. US 5,943,387 covers a sequential system for detection of biofilm preparation in laboratory conditions for use to evaluate the level of free particle movement in the in vitro bacteria culture, the structure of which is equipped with a medium container (containers) and a source producing an electric, electromagnetic, or magnetic effect in the microorganism suspension.

Another structural solution for the biofilm production device offers detection of microorganisms in *in situ* conditions for analysis with atomic force microscopy (AFM).

The invention aims at development of a container for production of a biofilm on a carbon carrier in the environment of the microbiological growth medium, allowing and ensuring uninterrupted measurement during analysis of the biofilm sample subjected to SEM analysis with potential to use the container as a reservoir for liquid or semi-liquid culture medium and allow for its change. Another objective is a development of an integrated container supporting biofilm production for qualitative and quantitative analysis in scope of studies of the microorganisms composing the biofilm of various growth requirements to ensure universal application in various fields of clinical and experimental microbiology.

The invention solves this problem by providing a compartment container to grow microorganisms and produce biofilm for SEM analysis, which possess a vertical cylinder structure with a wider base and standard cork closing. The container is composed of two attached compartments, top and bottom, connected with a bushing. The bottom container is removable together with a stabilising ring serving as the base for the carbon disc for biofilm growith and possess a valve used to remove liquid medium. The container is supported on a base. The top and bottom compartments compose the environment of the liquid substance (microbiological growth medium). The medium removal level can be adjusted with the valve. This system ensures stable cultivation of the biofilm form in in vitro conditions. The side surface of the stabilising ring should have peripheral supports to allow its wedging in inside of the bottom container compartment. The container structure valve should be located no more than 2 mm above the base of the bottom compartment. The ring (4), stabilising the biofilm carrier in a form of a carbon disc , should be located near or at the height of the top edge of the bottom container compartment. The diameter of the ring stabilising the carbon disc should not exceed 10 mm. The minimum ratio between the diameter of the ring stabilising the biofilm carrier and the container diameter should be 1:1.2. Preferably, the cross section of the stabilising ring is circular. The container base should ensure its stable vertical positioning, i.e. its diameter should be bigger than that of the container. The minimum ratio between the heights of the bottom and top compartment should be 0.5:1; the height of the bottom compartment should not be less than 20 mm and the height of the top compartment should not be less than 40 mm. The connecting bushing should be made of synthetic, polymer, etc. material, e.g. silicone or natural rubber. The container should be made of a synthetic material or glass. The stabilising ring must provide actual conduction for transfer of electrons from the SEM emitter.

The invention allows for use of any liquid medium as an environment for microorganism growth, simultaneously facilitating separation of the bottom compartment and biofilm on the carbon carrier regardless of the qualitative and quantitative biofilm content. As a universal tool, it can be used to study bacterial and fungal biofilm. The carbon carrier in the container allows for growth of diverse biofilm on specific growing medium.

The invention reduces the total time required to prepare the biofilm sample for SEM analysis, limits the preparation activity of the personnel, and eliminates the risk of damage to the biofilm sample resulting from microorganism growth on a highly conductive medium.

Since the optimal size of a round container's diameter is between 10 mm and 20 mm and with consideration of the liquid substance volume required for the culture grown in the container, the minimum height of the whole container according to the invention should fall between 70 mm and 80 mm, depending on the type of the microbiological culture and purpose of the container. The container diameter should be between 10 mm and 15 mm and the maximum ring diameter should be between 8 and 13 mm.

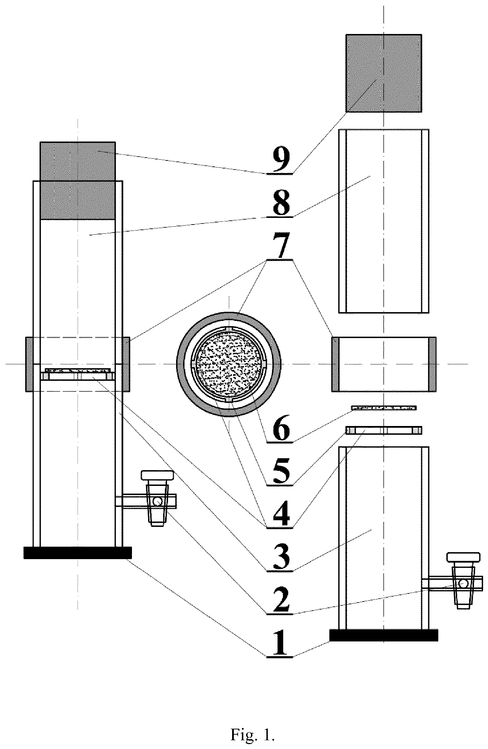

The invention is presented in production examples in Figure 1, which is a side view schematic of the container and the container's components.

### Example I

The glass compartment container for growing microorganisms and producing biofilm for SEM analysis has a vertical cylinder structure with a wider base and standard cork closing (9). The container is composed of two attached compartments, top (8) and bottom (3), connected with a silicone bushing (7). The bottom container (3) is removable together with the ring (4) made of conducting material and a stabilising the carbon disc (6) for biofilm growth and has a valve (2) used to remove liquid medium. The compartment container is supported on a bearing base (1). The top (8) and bottom (3) compartments of the container compose the environment of the liquid substance (microbiological medium). The medium removal level can be adjusted with a valve (2). This system ensures stable cultivation of the biofilm form in in vitro conditions. The side surface of the stabilising ring should have peripheral supports (5) to allow its wedging in the inside of the bottom container compartment (3). The valve (2) is located 2 mm above the bottom compartment (3) base. The ring (4) stabilising the biofilm carrier in form of a carbon disc (6) - is located at the height of the top edge of the bottom container compartment (3). The diameter of the ring (4) stabilising the carbon disc does not exceed 10 mm.

The minimum ratio between the diameter of the ring (4) stabilising the biofilm carrier and the container diameter should be 1:1.2. The cross section of the stabilising ring (4) is circular and the diameter of the container's (1) bearing base is bigger than that of the container. The proportion between the heights of the compartments (3) and (8) is 0.5:1.

### Example II

The synthetic compartment container for growing microorganisms and producing biofilm for SEM analysis has a vertical cylinder structure with a wider base and standard cork closing (9). The container is composed of two attached compartments, top (8) and bottom (3), connected with a silicone bushing (7). The bottom container (3) is removable together with the ring (4) made of conducting material and serving as the bearing base of the carbon disc (6) for biofilm growing and has a valve (2) used to remove liquid medium. The compartment container is supported on a bearing base (1). The top (8) and bottom (3) compartments of the container generally compose the environment of the liquid substance (microbiological medium). The medium removal level can be adjusted with the valve (2). This system ensures stable cultivation of the biofilm form in in vitro conditions. The valve (2) is located 1.5 mm above the bottom compartment (3). The ring (4) stabilising the biofilm carrier in form of a carbon disc (6) is be located near the height of the top edge of the bottom container compartment (3). The diameter of the ring (4) stabilising the carbon disc does not exceed 8 mm.

The minimum ratio between the diameter of ring (4) stabilising the biofilm carrier and the container diameter is 1:1.4. The cross section of the stabilising ring (4) is circular and the diameter of the container's (1) bearing base is bigger than that of the container. The ratio between the heights of the compartments (3) and (8) is 0.6:1.1.

## Claims

1. Container to produce microorganism biofilm on a carbon carrier **characterised in that** it possesses a removable top (8) and bottom (3) compartments, connected with a movable bushing (7), a stabilising ring (4) located in the bottom compartment (3) made of conductive material to serve as the bearing base of the carbon disc (6), a valve (2) for removal of liquid medium located in the bottom compartment (3), and closure (9) of the top compartment (8),

2. Container according to claim 1 **characterised in that** it possesses a base (1).

3. Container according to claim 2 **characterised in that** the diameter of the base (1) is bigger than the diameter of the bottom container compartment (3).

4. Container according to claim 1 or 2 or 3, **characterised in that** the side surface of the stabilising ring (4) possess at least four supports (5) located symmetrically in a 90-degree range allowing to wedge the ring in the inside of the bottom container compartment (3).

5. Container according to claim 1 or 2 or 3 or 4, **characterised in that** the stabilising ring (4) is installed at the height of the bottom compartment's edge (3).

6. Container according to claim 1 or 2 or 3 or 4 or 5, **characterised in that** the valve (2) is located no more than 2 mm above the bottom container compartment (3) base.

7. Container according to claim 1 or 2 or 3 or 4 or 5 or 6, **characterised in that** the stabilising ring (4) for stabilisation of the carbon disc has a diameter not larger than 10 mm.

8. Container according to claim 1 or 2 or 3 or 4 or 5 or 6 or 7, **characterised in that** the ratio between the diameter of the stabilising ring (4) of the biofilm carrier to the diameter of the container is at least 1:1.2

9. Container according to claim 1 or 2 or 3 or 4 or 5 or 6 or 7 or 8, **characterised in that** the diameter of the stabilising ring (4) stabilising the carbon disc (6) is at least 1 mm smaller than the diameter of the container and said ring is movably integrated with the internal wall of the bottom container (3).

10. Container according to claim 1 or 2 or 3 or 4 or 5 or 6 or 7 or 8 or 9, **characterised in that** the cross section of the stabilising ring (4) is circular.

11. Container according to claim 1 or 2 or 3 or 4 or 5 or 6 or 7 or 8 or 9 or 10, **characterised in that** the ratio between the heights of compartments (3) and (8) is at least 0.5:1 and wherein the height of the bottom compartment is greater than or equal to 20 mm and the height of the top compartment is greater than or equal to 40 mm.

12. Container according to claim 1 or 2 or 3 or 4 or 5 or 6 or 7 or 8 or 9 or 10 or 11, **characterised in that** the connecting bushing (7) is made of polymer.

13. Container according to claim 12, **characterised in that** the connecting bushing (7) is made of silicone or natural rubber.

14. Container according to claim 1 or 2 or 3 or 4 or 5 or 6 or 7 or 8 or 9 or 10 or 11 or 12 or 13, **characterised in that** the diameter of the container with a circular cross-section is between 10 mm and 20 mm and minimum height of the entire container is between 70 mm and 80 mm, depending on the type of microbiological culture and application of the container.

15. Container according to claim 1 or 2 or 3 or 4 or 5 or 6 or 7 or 8 or 9 or 10 or 11 or 12 or 13 or 14, **characterised in that** the diameter of the container is between 10 mm and 15 mm and wherein the maximum diameter of the container without the base is between 8 and 13 mm.

## Patentansprüche

1. Behälter zur Herstellung eines Biofilms aus Mikroorganismen auf einem Kohlenstoffträger, **dadurch gekennzeichnet, dass** er eine abnehmbare obere (8) und untere (3) Fächer, die mit einer beweglichen Buchse (7) verbunden sind, einen Stabilisierungsring (4), der sich in dem unteren Fach (3) befindet und aus leitfähigem Material besteht, um als Auflagefläche für die Kohlenstoffscheibe (6) zu dienen, ein Ventil (2) zur Entnahme des flüssigen Mediums, das sich in der unteren Kammer (3) befindet, und einen Verschluss (9) des oberen Fachs (8) aufweist.

2. Behälter nach Anspruch 1, **dadurch gekennzeichnet, dass** er einen Boden (1) aufweist.

3. Behälter nach Anspruch 2, **dadurch gekennzeichnet, dass** der Durchmesser des Bodens (1) größer ist als der Durchmesser des unteren Behälterfachs (3).

4. Behälter nach Anspruch 1 oder 2 oder 3, **dadurch gekennzeichnet, dass** die Seitenfläche des Stabilisierungsrings (4) mindestens vier symmetrisch in einem 90-Grad-Bereich angeordnete Stützen (5) aufweist, die es ermöglichen, den Ring im Inneren des unteren Behälterfachs (3) zu verkeilen.

5. Behälter nach Anspruch 1 oder 2 oder 3 oder 4, **dadurch gekennzeichnet, dass** der Stabilisierungsring (4) auf der Höhe des unteren Fachrandes (3) angebracht ist.

6. Behälter nach Anspruch 1 oder 2 oder 3 oder 4 oder 5, **dadurch gekennzeichnet, dass** das Ventil (2) nicht mehr als 2 mm über dem Boden des Behälterfachs (3) angeordnet ist.

7. Behälter nach Anspruch 1 oder 2 oder 3 oder 4 oder 5 oder 6, **dadurch gekennzeichnet, dass** der Stabilisierungsring (4) zur Stabilisierung der Kohlescheibe einen Durchmesser von nicht mehr als 10 mm aufweist.

8. Behälter nach Anspruch 1 oder 2 oder 3 oder 4 oder 5 oder 6 oder 7, **dadurch gekennzeichnet, dass** das Verhältnis zwischen dem Durchmesser des Stabilisierungsrings (4) des Biofilmträgers und dem Durchmesser des Behälters mindestens 1:1,2 beträgt.

9. Behälter nach Anspruch 1 oder 2 oder 3 oder 4 oder 5 oder 6 oder 7 oder 8, **dadurch gekennzeichnet, dass** der Durchmesser des die Kohlescheibe (6) stabilisierenden Rings (4) mindestens 1 mm kleiner ist als der Durchmesser des Behälters und der Ring beweglich in die Innenwand des Bodenbehälters (3) integriert ist.

10. Behälter nach Anspruch 1 oder 2 oder 3 oder 4 oder 5 oder 6 oder 7 oder 8 oder 9, **dadurch gekennzeichnet, dass** der Querschnitt des Stabilisierungsrings (4) kreisförmig ist.

11. Behälter nach Anspruch 1 oder 2 oder 3 oder 4 oder 5 oder 6 oder 7 oder 8 oder 9 oder 10, **dadurch gekennzeichnet, dass** das Verhältnis zwischen den Höhen der Fächer (3) und (8) mindestens 0,5:1 beträgt und wobei die Höhe des unteren Fachs größer oder gleich 20 mm und die Höhe des oberen Fachs größer oder gleich 40 mm ist.

12. Behälter nach Anspruch 1 oder 2 oder 3 oder 4 oder 5 oder 6 oder 7 oder 8 oder 9 oder 10 oder 11, **dadurch gekennzeichnet, dass** die Anschlussbuchse (7) aus Polymer besteht.

13. Behälter nach Anspruch 12, **dadurch gekennzeichnet, dass** die Anschlussbuchse (7) aus Silikon oder Naturkautschuk hergestellt ist.

14. Behälter nach Anspruch 1 oder 2 oder 3 oder 4 oder 5 oder 6 oder 7 oder 8 oder 9 oder 10 oder 11 oder 12 oder 13, **dadurch gekennzeichnet, dass** der Durchmesser des Behälters mit kreisförmigem Querschnitt zwischen 10 mm und 20 mm und die Mindesthöhe des gesamten Behälters zwischen 70 mm und 80 mm beträgt, je nach Art der mikrobiologischen Kultur und Anwendung des Behälters.

15. Behälter nach Anspruch 1 oder 2 oder 3 oder 4 oder 5 oder 6 oder 7 oder 8 oder 9 oder 10 oder 11 oder 12 oder 13 oder 14, **dadurch gekennzeichnet, dass** der Durchmesser des Behälters zwischen 10 mm und 15 mm liegt und wobei der maximale Durchmesser des Behälters ohne den Boden zwischen 8 und 13 mm liegt.

## Revendications

1. Récipient pour produire un biofilm de micro-organismes sur un support en carbone **caractérisé en ce qu'il** possède un compartiment supérieur (8) et inférieur (3) amovibles, reliés à une douille mobile (7), un anneau de stabilisation (4) située dans le compartiment inférieur (3) en matériau conducteur pour servir de base d'appui du disque de carbone (6), une vanne (2) pour l'évacuation du milieu liquide située dans le compartiment inférieur (3), et la fermeture (9) du compartiment supérieur (8).

2. Récipient selon la revendication 1 **caractérisé en ce qu'il** possède un fond (1).

3. Récipient selon la revendication 2, **caractérisé en ce que** le diamètre du fond (1) est supérieur au diamètre du compartiment inférieur (3) du récipient.

4. Récipient selon la revendication 1 ou 2, ou 3, **caractérisé en ce que** la surface latérale de l'anneau de stabilisation (4) possède au moins quatre supports (5) situés symétriquement dans une plage de 90 degrés permettant de caler l'anneau à l'intérieur du compartiment inférieur du récipient (3).

5. Récipient selon la revendication 1 ou 2, ou 3, ou 4, **caractérisé en ce que** l'anneau de stabilisation (4) est installé à hauteur du bord du compartiment inférieur (3).

6. Récipient selon la revendication 1 ou 2, ou 3, ou 4, ou 5, **caractérisé en ce que** la vanne (2) est située au plus à 2 mm au-dessus du fond du compartiment inférieur (3) du récipient.

7. Récipient selon la revendication 1 ou 2, ou 3, ou 4, ou 5, ou 6, **caractérisé en ce que** l'anneau de stabilisation (4) pour la stabilisation du disque de carbone a un diamètre non supérieur à 10 mm.

8. Récipient selon la revendication 1 ou 2, ou 3, ou 4, ou 5, ou 6, ou 7, **caractérisé en ce que** le rapport entre le diamètre de l'anneau de stabilisation (4) du support de biofilm et le diamètre du récipient est d'au moins 1:1,2.

9. Récipient selon la revendication 1 ou 2, ou 3, ou 4, ou 5, ou 6, ou 7, ou 8, **caractérisé en ce que** le diamètre de l'anneau de stabilisation (4) stabilisant le disque de carbone (6) est au moins 1 mm inférieur au diamètre du récipient et ledit anneau est intégré de manière mobile à la paroi interne du récipient inférieur (3).

10. Récipient selon la revendication 1 ou 2, ou 3, ou 4, ou 5, ou 6, ou 7, ou 8, ou 9, **caractérisé en ce que** la section transversale de l'anneau de stabilisation (4) est circulaire.

11. Récipient selon la revendication 1 ou 2, ou 3, ou 4, ou 5, ou 6, ou 7, ou 8, ou 9, ou 10, **caractérisé en ce que** le rapport entre les hauteurs des compartiments (3) et (8) est d'au moins 0,5:1 et dans lequel la hauteur du compartiment inférieur est supérieure ou égale à 20 mm et la hauteur du compartiment supérieur est supérieure ou égale à 40 mm.

12. Récipient selon la revendication 1 ou 2, ou 3, ou 4, ou 5, ou 6, ou 7, ou 8, ou 9, ou 10, ou 11, **caractérisé en ce que** la douille de raccordement (7) est en polymère.

13. Récipient selon la revendication 12, **caractérisé en ce que** la douille de raccordement (7) est en silicone ou en caoutchouc naturel.

14. Récipient selon la revendication 1 ou 2, ou 3, ou 4, ou 5, ou 6, ou 7, ou 8, ou 9, ou 10, ou 11, ou 12, ou 13, **caractérisé en ce que** le diamètre du récipient à section circulaire est compris entre 10 mm et 20 mm et la hauteur minimale de l'ensemble du récipient est comprise entre 70 mm et 80 mm, selon le type de culture microbiologique et l'application du récipient.

15. Récipient selon la revendication 1 ou 2, ou 3, ou 4, ou 5, ou 6, ou 7, ou 8, ou 9, ou 10, ou 11, ou 12, ou 13, ou 14, **caractérisé en ce que** le diamètre du récipient est compris entre 10 mm et 15 mm et dans lequel le le diamètre maximum du récipient sans le fond est compris entre 8 et 13 mm.
